# EUROPEAN PATENT APPLICATION

(11) **EP 2 666 405 A1**
(43) Date of publication of application: **27.11.2013**
(21) Application number: 13168435.9
(22) Date of filing: 20.05.2013
(51) Int. Cl.: A61B 5/00

(54) **Pressure ulcer detection systems**

(30) Priority: 21.05.2012 US 201261649482 P; 11.09.2012 US 201213609776
(71) Applicant: Hill-Rom Services, Inc., Batesville, IN 47006-9167 (US)
(72) Inventor: Ribble, David, Indianapolis, IN 46202 (US); Lachenbruch, Charles A, Lakeway, TX 78734 (US)
(74) Representative: Findlay, Alice Rosemary

(57) **Abstract**

Pressure ulcer detection systems and related methods are disclosed. Images of a patient supported by a person support apparatus are captured and analyzed to identify areas of the patient's skin which have a color different from surrounding areas of the patient's skin by a predetermined amount. A dielectric material attached with a positively charged electrode and a negatively charged electrode is configured to be in contact with the patient's skin. Differences in electric potential between areas of skin relative to surrounding areas of skin are identified.

## Description

Detection of onset and /or development of pressure sores remain an ongoing challenge. Since pressure sores tend to develop in bedridden patients with limited mobility, detection of symptoms and monitoring of sores is particularly difficult. While several systems exist to detect and monitor pressure sore development a need exists to continue development in this area.

The present disclosure includes one or more of the the following features alone or in any combination.

One embodiment of a system for detecting a pressure ulcer for use with a person support apparatus comprises a system for detecting a pressure ulcer for use with a person support apparatus, comprising an image capture device, said image capture device configured to capture an image of at least a portion of a person supported by the person support apparatus; a processor configured to process information from the captured images and to communicate with a destination in response to image variation or lack thereof..

The image capture device may be configured to communicate with a controller which forms part of a caregiver interface configured to determine variations in light intensity and the caregiver interface may be configured to control at least one function of a person support apparatus.

Another embodiment of a system for detection a pressure ulcer for use with a person support apparatus comprises at least one optical fiber. A portion of the optical fiber is configured to be embedded in a person support surface, the optical fiber configured to at least one of supply and capture light through a transparent region in the person support surface. An optical generator is configured to supply light to the at least one optical fiber and is configured to communicate with a caregiver interface.

Another embodiment of a system for detecting a pressure ulcer for use with a person support apparatus comprises a person support surface comprising an outer surface configured to support a person thereon, said outer surface comprising a dielectric material. An electrode configured to be positively charged and another electrode configured to be negatively charged are attached to the dielectric material. A battery is configured to provide an electric potential between the electrodes. A caregiver interface configured to measure variation in electrical potential between the positively charged electrode and negatively charged electrode.

One method for detecting a pressure ulcer is disclosed which comprises capturing an image of at least a portion of a person supported by a person support apparatus, transmitting the image to a caregiver interface, determining variations in intensity of at least one region in the image with respect to a surrounding region in the image and alerting a caregiver in intensity of at least one region in the image is greater than a predetermined threshold with respect to the surrounding region.

Another method for detecting a pressure ulcer is disclosed which comprises measuring change in the electric field in a dielectric material of a person support surface, transmitting the change in electric field to a caregiver interface, determining variation in electric field of at least one region with respect to a surrounding region and alerting a caregiver if the variation in electric field is greater than a predetermined threshold.

The invention will now be further described by way of example with reference to the accompanying drawings, in which:

FIG. 1 is a block diagram of a system for detecting pressure sores for use with a person support apparatus wherein an image capture device communicates with a caregiver interface, constructed according to one or more of the principles disclosed herein;

FIG. 2 is a block diagram of another system for detecting pressure sores wherein at least one optical fiber is housed within a mattress topper, constructed according to one or more of the principles disclosed herein;

FIG. 3A is a depiction of a concentric construction of an optical fiber for use in a system for detecting pressure sores, constructed according to one or more of the principles disclosed herein;

FIG. 3B is a depiction of a another construction of an optical fiber for use in a system for detecting pressure sores, constructed according to one or more of the principles disclosed herein;

FIG. 4 is a block diagram of a system for detecting pressure sores for use with a person support apparatus wherein the surface of a person support surface comprises a dielectric material, constructed according to one or more of the principles disclosed herein;

FIG. 5 is a depiction of construction and use of a dielectric material in a system for detecting pressure sores, constructed according to one or more of the principles disclosed herein;

FIG. 6 is a block diagram of a system for detecting pressure sores for use with a person support apparatus wherein the caregiver interface comprises a controller, a display device and a caregiver input device, constructed according to one or more of the principles disclosed herein;

It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be briefly mentioned or omitted so as to not unnecessarily obscure the described embodiments. The examples used herein are intended merely to facilitate an understanding of ways in which embodiments may be practiced and to further enable those of skill in the art to practice the embodiments. Accordingly, the examples and embodiments herein are merely illustrative. Moreover, it is noted that like reference numerals represent similar parts throughout the several views of the drawings.

It is understood that the subject matter claimed is not limited to the particular methodology, protocols, devices, apparatus, materials, applications, etc., described herein, as these may vary. It is also to be understood that the terminology used herein is used for the purpose of describing particular embodiments only.

Unless defined otherwise, all technical and scientific terms used herein have the same meanings as commonly understood by one of ordinary skill in the art.

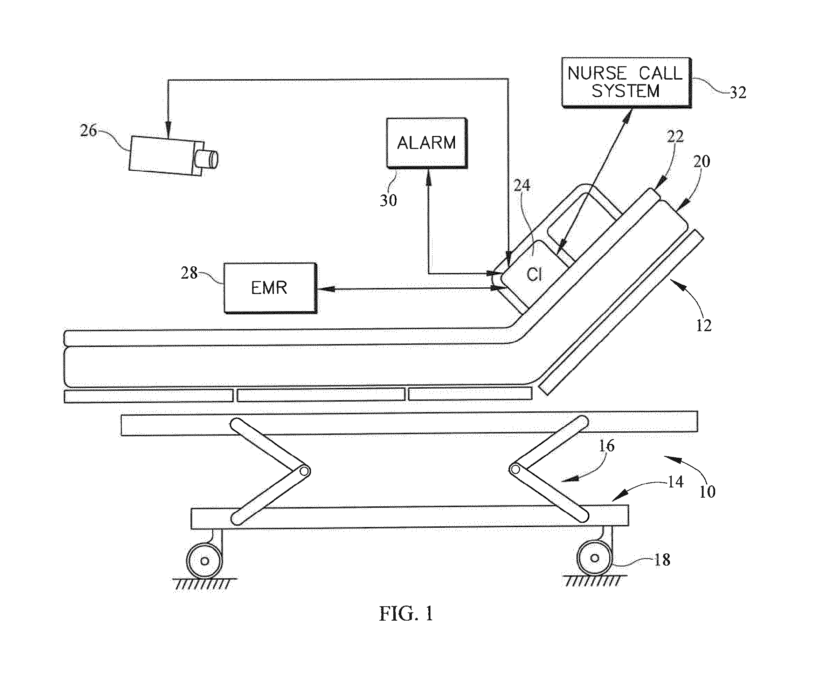

One embodiment of a system to detect pressure ulcers on a person supported by a person support apparatus 10 is shown in FIG. 1. In this embodiment, the person support apparatus 10 is a bed, however, in other embodiments the person support apparatus 10 may be a wheelchair, stretcher or any other apparatus configured to support a person thereon. The person support apparatus 10 in this embodiment comprises an upper frame 12 which is supported over a lower frame 14 by supports 10. The upper frame 12 comprises one of more sections and the support 16 is configured to variably elevate at least one section of the upper frame 12 with respect to the lower frame 14. The lower frame 14 rests on at least one caster wheel 18 in this embodiment, allowing the person supported apparatus 10 to be transported. A person support surface 20 rests on the person support apparatus 10. In this embodiment the person support surface 20 is a mattress and comprises fluid filled bladders, in other embodiments the person support surface may be made of any combination of bladders, foam and other polymeric materials. A mattress topper 22 is configured to be positioned on top of the person support surface 20 such that a person can be supported on top of the mattress topper 22. An image capture device 26 is configured to capture images of a person supported by the mattress topper 22. The image capture device 26 is configured to capture electromagnetic radiation and in this embodiment is configured to capture the near infra-red region of the electromagnetic spectrum (wavelength in the range of 800 nanometers - 2500 nanometers). In other embodiments, the image capture device 26 may be configured to capture any portion of the electromagnetic spectrum. The image capture device 26 is configured to transmit data to a controller in the form of a caregiver interface (Cl) 24. In this embodiment, the caregiver interface (Cl) 24 comprises a memory 44 to store information for a predetermined time, a processor (not shown) to process information and a display (not shown). Memory 44 may be of any type including volatile and non-volatile. The Cl 24 is configured to control at least one function of the person support apparatus 10 in this embodiment, while in other embodiments a system to detect pressure ulcers comprises a dedicated Cl 24. The Cl 24 is configured to communicate with an alarm 30. Alarm 30 is an audio alert in this embodiment, in other embodiments alarm 30 may be any combination of audio, visual and / or tactile alerting system. The Cl 24 is configured to communicate with a nurse call system 32 configured to alert a caregiver. The Cl 24 is also configured to communicate with an electronic medical records (EMR) database 28.

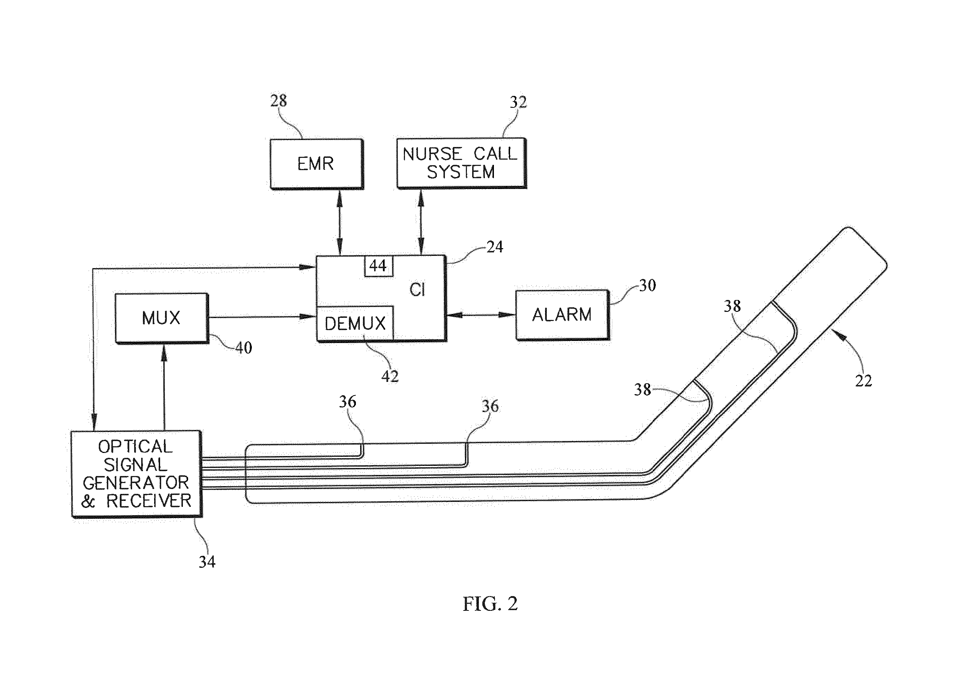

During operation, upon receiving data from the image capture device 26 the Cl 24 processes this information to identify regions of the image or various regions in multiple images wherein difference in color of up to 25 percent is identified with respect to a surrounding region in this embodiment. In another embodiment the difference in color is compared relative to an average value assigned to the color of skin of the patient. In another embodiment a caregiver sets the percentage difference in color that the Cl 24 uses as a threshold to identify areas of the skin with color difference. The Cl 24 also has the capability to identify the patient supported by the mattress topper 22. Cl 24 is therefore configured to distinguish areas of the patient's skin which demonstrate a difference in color above a predetermined threshold relative to adjacent areas of the patient's skin. Upon making a determination that certain areas of the patient's skin show a difference in coloration greater than the threshold, the Cl 24 activates the alarm 30. In this embodiment the Cl 24 also communicates with a nurse call system 32 upon making the aforementioned determination. The Cl 24 also communicates with an EMR 28 database and in this embodiment, upon making the determination that certain areas of the patient's skin show a difference in coloration greater than the threshold the Cl 24 conveys information related to the area of the patient's skin where this determination has been made and /or the difference in color of the skin in terms of percentage or a value. In one embodiment shown in FIG. 2 the Cl 24 comprises a memory 44 and the Cl 24 stores location of areas of the patient's skin which are determined to have a color differing greater than the predetermined threshold. In this embodiment the Cl 24 is configured to compare similar areas of a patient's skin from images taken by the image capture device 26 over a period of time. The Cl 24 is configured to determine the progression in terms of color change over time of an area of the skin identified to be of interest. In this embodiment if the area of the skin identified to be of interest does not show a color change over time, the Cl 24 assigns those areas as permanent area of discoloration indicative of potential birth marks or other skin conditions. In one embodiment the areas of skin identified as permanent area of discoloration are assigned flag indicating that the color in these areas is to be normalized with respect to other areas of the skin for assessment of skin discoloration. In one embodiment the areas of skin identified as permanent area of discoloration are weighted appropriately in the images captured by the image capture device 26. In another embodiment the patient is supported by the mattress 20 instead of the mattress topper 22.

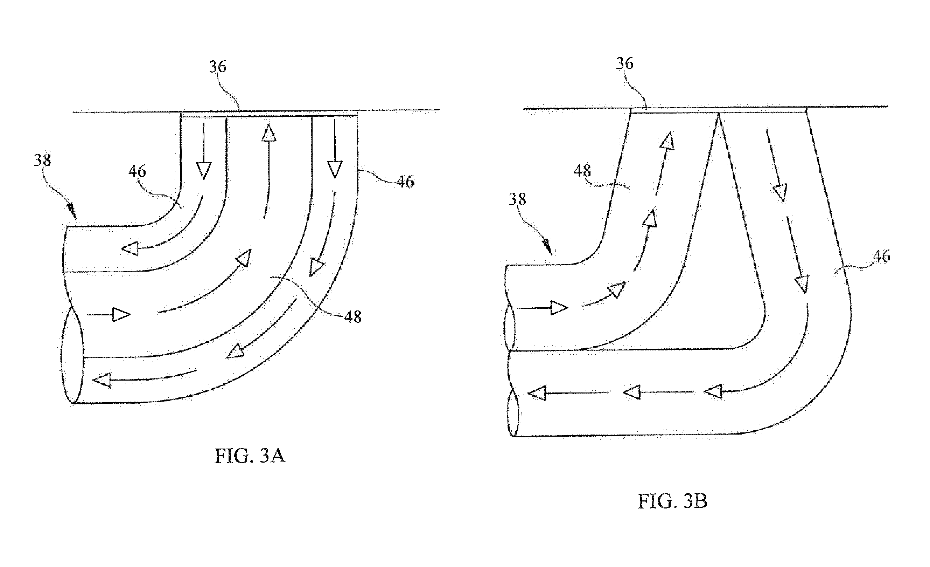

Another embodiment of a system to detect pressure ulcers on a person supported by a person support apparatus 10 is shown in FIG. 2. As shown in FIG. 2 at least one optical fiber 38 is embedded in the mattress topper 22 in this embodiment. In another embodiment the at least one optical fiber 38 is embedded in the mattress 20. One axial end of the optical fiber 38 is configured to emit a portion of the electromagnetic spectrum through a transparent region 36 of the mattress topper 22 on a person supported by the mattress topper 22 as seen in FIG. 2, FIG. 3A & FIG. 3B. The other axial end of the optical fiber 38 is supplied with a portion of the electromagnetic spectrum by an optical signal generator and receiver 34 as shown in FIG.2. The optical signal generator and receiver 34 is configured to communicate with Cl 24. The Cl 24 controls the optical signal generator and receiver 34 and provides the control signal to initiate transmission of a portion of electromagnetic spectrum through optical fiber 38 and the transparent region 36 onto the patient's skin. The optical fibers 38 also comprise the ability to receive light through the transparent region 36 and transmit this reflected light to the optical signal generator and receiver 34 in this embodiment. The optical signal generator and receiver 34 transmits light received from the optical fibers 38 to a multiplexer (MUX) 40 to multiplex signals received from more than one optical fibers 38 in this embodiment. The MUX 40 is configured to communicate with a demultiplexer (DEMUX) 42 housed in the Cl 24. The Cl 24 comprises a memory 44 in this embodiment. The Cl 24 is configured to communicate with an EMR 28, a nurse call system 32 and an alarm 30 in this embodiment.

In operation the Cl 24 provides a control signal to the optical signal generator and receiver 34 to transmit at least a portion of the electromagnetic spectrum through the optical fibers 38. Light reflected from the patient supported by the mattress topper 22 is captured by the optical fibers 38 and transmitted to the optical signal generator and receiver 34. The optical signal generator and receiver 34 transmits light received from the optical fibers 38 to the MUX 40 in this embodiment. In this embodiment the MUX 40 is a standalone device, while in another embodiment the MUX 40 may be housed within the optical signal generator and receiver 34. The MUX 40 multiplexes signals received from the various optical fibers 38 and transmits them to a DEMUX 42. The DEMUX 42 is housed within the Cl 42 in this embodiment, in another embodiment the DEMUX 42 is a standalone device in communication with the Cl 42. The DEMUX 42 separates out the signal from each optical fiber 38 and supplies the individual signals to a processor in the Cl 42 in this embodiment. In another embodiment the multiplexed signal sent by the MUX 40 is logically de-multiplexed by the Cl 42 instead of using a physical DEMUX 42. In one embodiment the signal received through the transparent regions 36 is transmitted by the optical fibers 38 directly to the MUX 40 instead of going through the optical signal generator and receiver. The Cl 42 comprises signal conditioning functionality in one embodiment to condition signals received through the optical fibers 38.

FIG. 3A and FIG. 3B show two embodiments of optical fiber construction for use in a system to detect pressure ulcers on a person supported by a person support apparatus 10 as shown in FIG. 2. FIG. 3A shows co-axially constructed optical fibers 38 wherein the light supplying fiber 48 transmits light through the transparent region 36 onto the skin of the person supported by the mattress topper 22. Light received through the transparent region 36 into the optical fibers 38 is carried through the light receiving fiber 46. FIG. 3B shows an optical fiber bundle 38 wherein the light supplying fiber 48 and the light receiving fiber 46 are arranged such that they occupy a portion of the transparent region 36 as shown in FIG. 3B. In FIG. 3A and FIG. 3B embodiments of optical fiber bundle construction are shown wherein the supplied and received signals are separated in different physical optical fibers. This aspires to prevent issues that may arise with internal reflections and refractions if a single optical fiber is used to both send and receive signals. In another embodiment a single optical fiber is used for both sending and receiving signals. The signals received from the single optical fiber are conditioned by the Cl 24 for reflection and refraction within the optical fiber 38, in one embodiment.

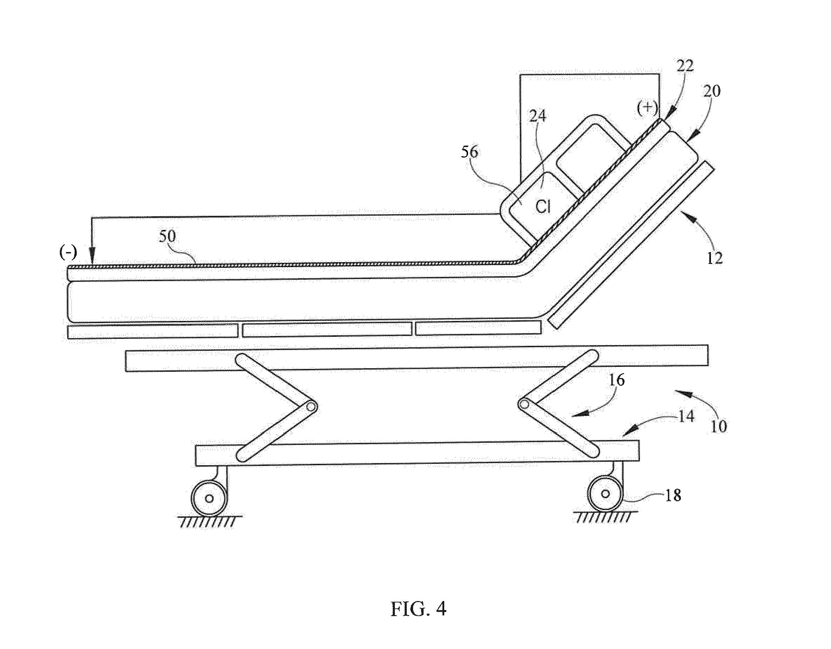

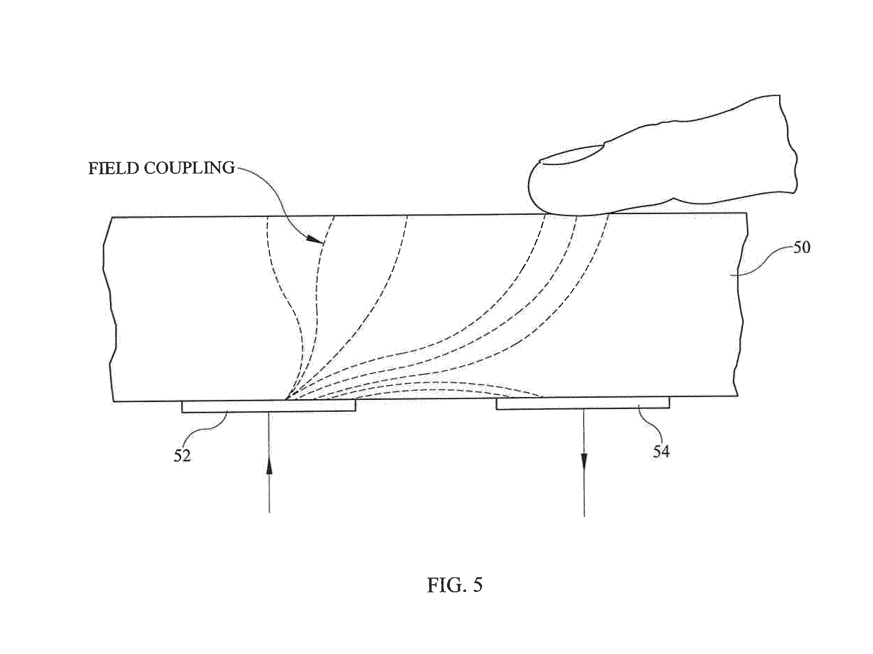

FIG. 4 shows one embodiment of a system to detect pressure ulcers on a person supported by a person support apparatus 10. As shown in FIG. 4 the mattress topper 22 comprises dielectric material 50 such that at least a portion of the dielectric material 50 is in contact with a person supported by the mattress topper 22. In another embodiment the dielectric material 50 may be incorporated in the mattress 20. In this embodiment the fibers of the surface of the mattress topper 22 that are configured to be in contact with the person are of a dielectric material 50. In another embodiment a layer of dielectric material 50 may be placed on top of the mattress topper 22. The dielectric material 50 is connected to a battery 56 which supplies an electric potential to electrodes coupled to the dielectric material as shown in FIG. 5. The Cl 24 is configured to measure variation in electrical potential between positively and negatively charged electrodes coupled to the dielectric material 50. In this embodiment the battery 56 is housed within the Cl 24.

FIG. 5 shows an embodiment of a system to detect pressure ulcers of FIG. 4 wherein an electric potential is supplied to a dielectric material 50 configured to be in contact with a person. At least one positively charged electrode 52 and at least one negatively charged electrode 54 are placed in contact with dielectric material 50. A person in contact with the dielectric material 50 causes variation in the electric potential between at least one positively charged electrode 52 and negatively charged electrode 54 due to field coupling. Furthermore the system to detect pressure ulcers is configured to distinguish between changes in electrical potential between positively and negatively charged electrodes when the dielectric material is in contact with skin susceptible to pressure ulcers and healthy skin. In one embodiment the Cl 24 is configured to measure the difference in potential between positively and negatively charged electrodes when the dielectric material is in contact with skin susceptible to pressure ulcers and healthy skin because skin susceptible to pressure ulcers retains fluid or displays Odeama. The presence of fluid in certain areas of skin allows the Cl 24 to differentiate between healthy skin and skin displaying Odeama. In one embodiment the dielectric material 50 comprises an array of positively charged electrodes 52 and negatively charged electrodes 54. The Cl 24 is configured to locate the local response of the patient's skin with respect to adjacent regions of the patient's skin based on change in potential between the various positively and negatively charged electrodes and identify areas of the patient's skin susceptible to pressure ulcers.

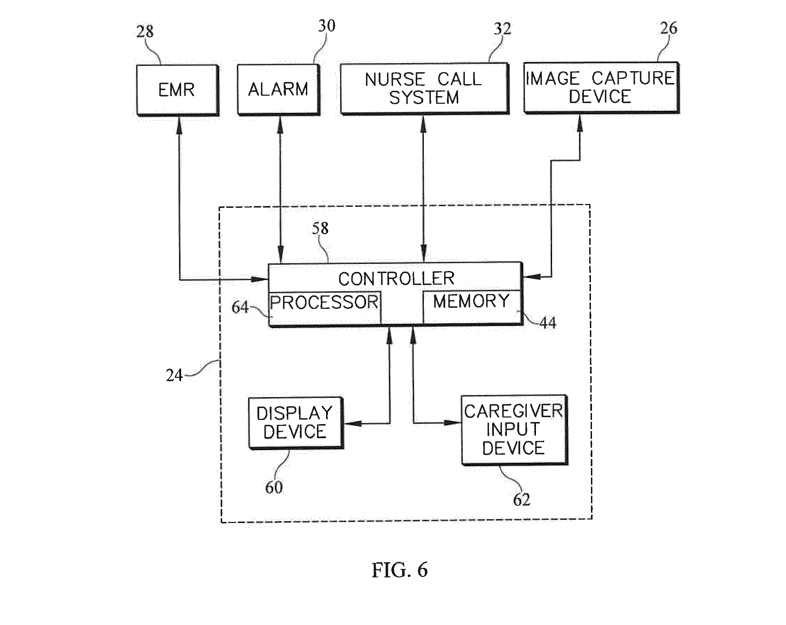

Another contemplated embodiment includes a caregiver interface (Cl) 24 including a controller 58 in communication with a display device 60 and caregiver input device 62, the controller 58 being a physically different device from the display device 60 and caregiver input device 62 in this embodiment as shown in FIG. 6. In this embodiment the controller 58 comprises a processor 64 and memory 44 in communication with a display device 60 and care giver input device 62. In this embodiment the display device 60 is a monitor and the care giver input device 62 is a pendant. The display device 60 and the caregiver input device 62 are portions of a touch sensitive screen in another embodiment.

The foregoing description is for the purpose of illustration only defined by the claims as set forth hereinafter together with any equivalents thereof entitled to. The use of any and all examples, or exemplary language (e.g., "such as") provided herein, is intended merely to better illustrate the subject matter. The use of the term "based on" and other like phrases indicating a condition for bringing about a result is not intended to foreclose any other conditions that bring about that result. No language in the specification should be construed as indicating any element as essential.

Preferred embodiments are described herein, including the best mode known to the inventor. Of course, variations of those preferred embodiments will become apparent to those of ordinary skill in the art upon reading the foregoing description. The inventor expects skilled artisans to employ such variations as appropriate. Moreover, any combination of the above-described elements in all possible variations thereof is encompassed unless otherwise indicated herein or otherwise clearly contradicted by context.
Embodiments of the invention can be described with reference to the following numbered clauses, with preferred features laid out in the dependent clauses:
1. A system for detecting a pressure ulcer for use with a person support apparatus, comprising:
   an image capture device, said image capture device configured to capture images of at least a portion of a person supported by a person support apparatus;
   a caregiver interface, said image capture device configured to communicate with said caregiver interface which is configured to determine variations in light intensity in at least one image of a person's skin, said caregiver interface configured to control at least one function of a person support apparatus.
2. The system of clause 1 wherein said caregiver interface is configured to determine variations in frequency of light in said at least one image captured by said image capture device.
3. The system of clause 1, wherein said caregiver interface is configured to communicate with a nurse call system.
4. The system of clause 1, wherein said caregiver interface is configured to communicate with an alarm.
5. The system of clause 1, wherein said caregiver interface is configured to communicate with an electronic medical records database.
6. The system of clause 1, wherein said caregiver interface comprises a controller.
7. The system of clause 1, wherein said caregiver interface comprises a display.
8. The system of clause 1, wherein said caregiver interface comprises a caregiver input device.
9. A system for detecting a pressure ulcer for use with a person support apparatus, comprising:
   at least one optical fiber;
   a person support surface configured to support a person thereon, at least a portion of said at least one optical fiber configured to be embedded in said person support surface, said optical fiber configured to at least one of supply and receive light through a transparent region in said person support surface;
   an optical signal generator configured to supply light to said at least one optical fiber;
   a caregiver interface, said optical signal generator configured to communicate with said caregiver interface.
10. The system of clause 9, wherein said caregiver interface is configured to communicate with a nurse call system.
11. The system of clause 9, wherein said caregiver interface is configured to communicate with an alarm.
12. The system of clause 9, wherein said caregiver interface is configured to communicate with an electronic medical records database.
13. The system of clause 9 wherein said optical signal generator is configured to receive light received by said at least one optical fiber.
14. The system of clause 13 further comprising a multiplexer, wherein said optical generator is configured to communicate information indicative of light received from said at least one optical fiber to said caregiver interface via said multiplexer.
15. The system of clause 14, wherein said caregiver interface comprises a demultiplexer configured to communicate with said multiplexer to receive information from said optical signal generator.
16. The system of clause 15 wherein said caregiver interface is configured to determine variations in light intensity received based on information received by said demultiplexer through said multiplexer.
17. The system of clause 16 further comprising a nurse call system, wherein said caregiver interface is configured to communicate with said nurse call system upon determination that said variation in light intensity exceeds a predetermined threshold.
18. The system of clause 9 wherein said caregiver interface further comprises a memory to store information received from said optical signal generator through said optical signal generator.
19. The system of clause 9 further comprising a multiplexer which receives light from more than one said optical fiber.
20. The system of clause 19 wherein said caregiver interface comprises a demultiplexer configured to communicate with said multiplexer.
21. The system of clause 19 wherein said caregiver interface further comprises a memory to store information received from said optical signal generator through said optical signal generator.
22. The system of clause 19 wherein said caregiver interface is configured to communicate with a nurse call system upon determination that said variation in light intensity exceeds a predetermined threshold.
23. The system of clause 9 wherein said caregiver interface receives information from at least said one optical fiber.
24. The system of clause 23 further comprising a nurse call system, wherein said caregiver interface is configured to communicate with said nurse call system upon determination that said variation in light intensity received from said at least optical fiber exceeds a predetermined threshold.
25. The system of clause 9 wherein said optical signal generator is housed within said caregiver interface.
26. The system of clause 9 wherein said optical fiber is of a co-axial construction.
27. The system of clause 9 wherein said person support surface is a mattress topper.
28. The system of clause 9, wherein said caregiver interface comprises a controller.
29. The system of clause 9, wherein said caregiver interface comprises a display.
30. The system of clause 9, wherein said caregiver interface comprises a caregiver input device.
31. A system for detecting a pressure ulcer for use with a person support apparatus, comprising:
   a person support surface comprising an outer surface configured to support a person thereon, said outer surface comprising dielectric material;
   at least one electrode configured to be positively charged, attached to said dielectric material;
   at least one electrode configured to be negatively charged, attached to said dielectric material;
   a battery configured to provide an electric potential between said at least one positively charged electrode and said at least one negatively charged electrode;
   a caregiver interface configured to measure variation in electrical potential between said at least one electrode configured to be positively charged and said at least one electrode configured to be negatively charged.
32. The system of clause 31 wherein said battery is housed in said caregiver interface.
33. A method for detecting a pressure ulcer comprising:
   capturing an image of at least a portion of a person supported by a person support apparatus;
   transmitting said image to a caregiver interface;
   determining variations in intensity of at least one region in the image with respect to a surrounding region in said image;
   alerting a caregiver if intensity of said at least one region in the image is greater than a predetermined threshold with respect to said surrounding region.
34. A method for detecting pressure ulcer comprising:
   measuring change in electric field in a dielectric material of a person support surface;
   transmitting said change in electric field to a caregiver interface;
   determining variation in electric field of at least one region with respect to a surrounding region;
   alerting a caregiver if the variation in electric field is greater than a predetermined threshold.

## Claims

1. A system for detecting a pressure ulcer for use with a person support apparatus, comprising:
an image capture device, said image capture device configured to capture an image of at least a portion of a person supported by the person support apparatus;
a processor configured to process information from the captured images and to communicate with a destination in response to image variation or lack thereof.

2. The system of claim 1 wherein the image variation is a variation in intensity of electromagnetic radiation.

3. The system of claim 1 wherein the image variation is a variation in frequency or wavelength of electromagnetic radiation.

4. The system of any preceding claim wherein the variation is a spatial variation in a single image.

5. The system of any one of claims 1 to 3 wherein the variation is a temporal variation.

6. The system of claim 5 wherein the information from the captured images is related to the person's skin coloration, and if the temporal variation does not exceed a threshold, the processor identifies the portion of the person captured in the image as an area of permanent discoloration.

7. The system of any one of claims 1 to 3 wherein the variation is a temporal variation of a spatial variation.

8. The system of either claim 4 or claim 7 wherein the spatial variation is of intensity in at least one region in the image with respect to another region in the image.

9. The system of any one of claims 1 to 7 wherein the variation is determined relative to a baseline, the baseline being a predetermined as representative of the skin of the person.

10. The system of any one of claims 1 to 7 wherein the processor compares the variation to a threshold variation, and wherein the communication to the destination is conditioned on the comparison.

11. The system of any one of claims 1 to 3 wherein the image capture device is a camera and the information from the captured images is related to skin color of the person and wherein the controller compares the image variation to a threshold variation.

12. The system of any preceding claim wherein the variation is a variation occurring substantially within a region of the electromagnetic spectrum from approximately 800 nanometers to 2500 nanometers.

13. The system of any preceding claim wherein the destination comprises an alarm.

14. The system of any preceding claim wherein the destination comprises a medical record.

15. The system of any preceding claim wherein the destination comprises a nurse call system.
